# EUROPEAN PATENT SPECIFICATION

(11) **EP 2 924 430 B1**
(45) Date of publication and mention of the grant of the patent: **14.08.2019**
(21) Application number: 15160793.4
(22) Date of filing: 25.03.2015
(51) Int. Cl.: G01N 33/00, G01N 27/12

(54) **GAS SENSOR PACKAGE**
GASSENSORPAKET
PAQUET DE CAPTEUR DE GAZ

(30) Priority: 25.03.2014 KR 20140034575
(43) Date of publication of application: 30.09.2015
(73) Proprietor: LG Innotek Co., Ltd., Seoul, 04637 (KR)
(72) Inventor: Hwang, Ji Hun, 100-714 Seoul (KR); Paik, Jee Heum, 100-714 Seoul (KR)
(74) Representative: Zardi, Marco

(56) References cited:
- JP-A- 2012 098 234
- US-A1- 2008 250 847
- US-A1- 2010 230 766
- US-A1- 2011 147 803
- MATTHIAS BUDDE ET AL: "The TECO Envboard: A mobile sensor platform for accurate urban sensing And more", NETWORKED SENSING SYSTEMS (INSS), 2012 NINTH INTERNATIONAL CONFERENCE ON, IEEE, 11 June 2012 (2012-06-11), pages 1-2, XP032206949, DOI: 10.1109/INSS.2012.6240573 ISBN: 978-1-4673-1784-9

## Description

### BACKGROUND OF THE INVENTION

### Field of the Invention

Embodiments of the present invention relate to a gas sensor package having high sensing efficiency and high connection reliability.

### Description of the Related Arts

A gas sensor is required to have characteristics, such as speed showing how fast the gas sensor can respond to a situation, sensibility which can respond to the detection of gas in spite of the detection of a small amount of the gas, durability showing how long the gas sensor can operate, economic efficiency showing that the sensor can be used by consumers without financial burdens, and the like.

In order for the gas sensor to be combined with an existing semiconductor process technology, the gas sensor should have characteristics of the easiness of integration and enumeration. A home gas leakage alarm made of tin oxide (SnO2) as a material has come into wide use as a practical gas sensor.

The gas sensor is divided into a semiconductor type using a change of resistance values according to a change in the amount of gas and an oscillator type using a change in an oscillation frequency generated when gas is absorbed onto an oscillator, which oscillates with a predetermined frequency. Most of the gas sensors have been used as the semiconductor type gas sensors having simple circuits and showing a stable thermal property at room temperature.

In general, a gas sensor has a package structure in which a gas sensing material or a sensing chip is mounted to the gas sensor, and should have a separate cap member for protecting an upper surface of the gas sensing material or the sensing chip, and a mesh-shaped member formed of minute nets is provided at an upper surface of the cap member so as to allow the ventilation of gas.

In this gas sensing package for sensing gas, a height of an upper structure is increased due to the cap member and the mesh-shaped member, and an entire size of the gas sensing package is further increased up to several times to dozens of times than that of a sensor chip because a wire bonding method is used when the sensor chip is connected to an electrode part. Due to this, there is a limit to implement miniaturization of the gas sensor. Examples of gas sensors and/or gas sensor packages according to the prior art are known from each of documents US2008/250847 A1, US2011/147803 A1, US2010/230766 A1 and JP2012098234 A.

### SUMMARY OF THE INVENTION

The present invention relates to a gas sensor package according to claim 1.

An aspect of embodiments as disclosed provides a gas sensor package in which a gas sensing element and a substrate are directly bonded by a flip chip bonding method so that a bonding wire can be removed, thereby enabling a reduction in a manufacturing cost and miniaturization of the gas sensor package.

Another aspect of embodiments as disclosed provides a gas sensor package that allows a reaction gas to sufficiently flow into a gas sensing element in a protective cap via a first ventilation hole and also allows the reaction gas in the protective cap to be discharged via a second ventilation hole so that efficiency ventilation can be performed, thereby simultaneously settling the problems of ventilation and radiant heat that may be generated due to the structural problem of a flip chip bonding method.

### BRIEF DESCRIPTION OF THE DRAWINGS

The accompanying drawings are included to provide a further understanding of the present invention, and are incorporated in and constitute a part of this specification. The drawings illustrate exemplary embodiments of the present invention and, together with the description, serve to explain principles of the present invention. In the drawings:
FIGS. 1 to 3 are views illustrated for explaining a gas sensor package according to an example;
FIGS. 4 and 5 are views illustrating a protective cap according to an example; and
FIGS. 6 to 11 are conceptual views illustrated for explaining a structure of a gas sensor package according to embodiments of the present invention.

### DETAILED DESCRIPTION OF THE INVENTION

Hereinafter, the configurations and operations according to embodiments of the present invention will be described in detail with reference to the accompanying drawings. The present invention may, however, be embodied in different forms and should not be construed as limited to the embodiments set forth herein. In the explanation with reference to the accompanying drawings, regardless of reference numerals of the drawings, like numbers refer to like elements through the specification, and repeated explanation thereon is omitted. Terms such as a first term and a second term may be used for explaining various constitutive elements, but the constitutive elements should not be limited to these terms. These terms is used only for the purpose for distinguishing a constitutive element from other constitutive element. As used herein, the singular forms are intended to include the plural forms as well, unless the context clearly indicates otherwise.

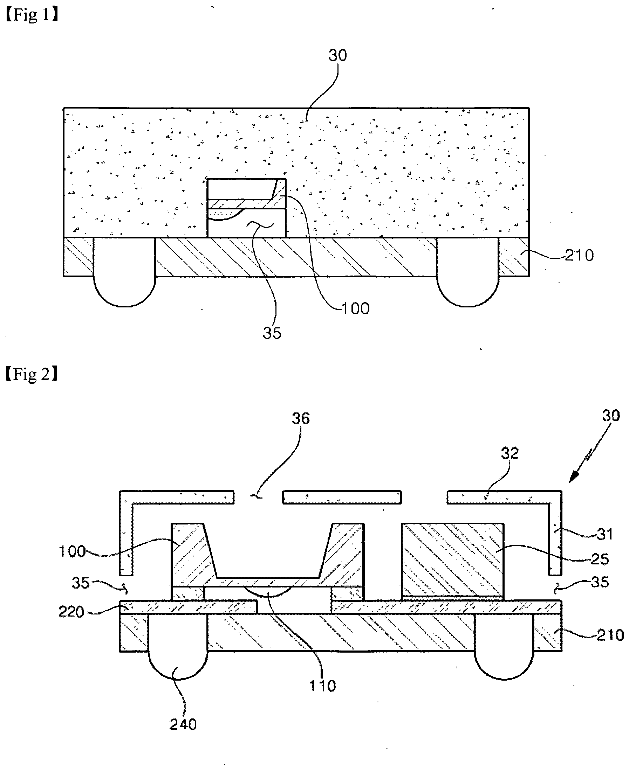

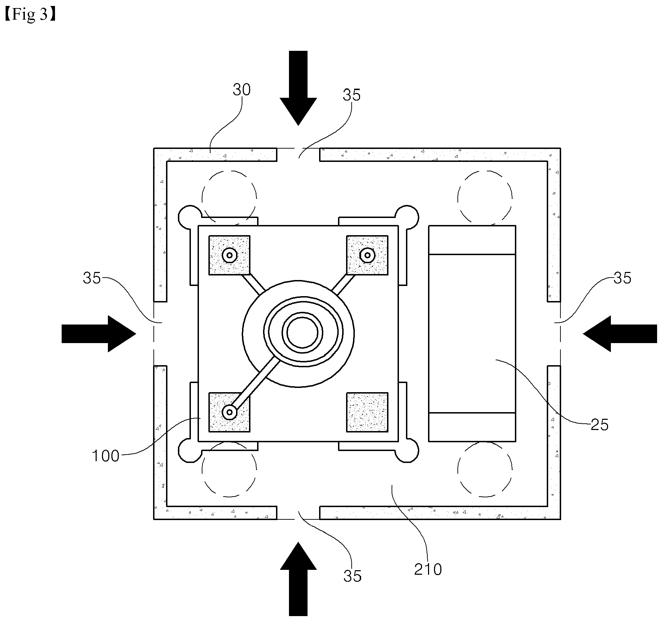

FIGS. 1 to 3 are views illustrated for explaining a gas sensor package according to an example namely, FIG. 1 is a side view of the gas sensor package according to the example, FIG. 2 is a side cross-sectional view of the gas sensor package according to the example, and FIG. 3 is an upper cross-sectional view of the gas sensor package according to the example.

The configuration of the gas sensor package according to the present example will be described with reference to FIGS. 1 to 3.

The gas sensor package according to the present example may include: a first substrate 210 including a metal pattern; a gas sensing element 100 mounted on the first substrate and having a gas sensing part disposed to face a surface of the first substrate 210; and protective cap 30 disposed on the first substrate 10, having a receiving space intended for receiving the gas sensing element 100, and including a first ventilation hole 35 passing through the receiving space and an exterior. In particular, with regard to the gas sensing element 100, a gas sensing part 110 is mounted to face the surface of the first substrate 210, namely, the gas sensing part being mounted in a flip chip bonding structure so that sensing efficiency can be increased. Furthermore, the protective cap 30 is implemented to enable gas to smoothly flow into the inside while protecting the gas sensing element 100, thereby increasing sensing efficiency.

More specifically, the gas sensing element 100 is a functional part including a sensing material which enables gas sensing, and all structures in a gas sensing type, which have been commonly commercialized, may be applied as the gas sensing element. That is, a sensing element using an oxide semiconductor, a sensing element using a carbon nanotube, various other sensing semiconductor chips, and the like may be applied as the gas sensing element. As a characteristic in the present embodiment of the invention, such a gas sensing element 100 is mounted on the first substrate to face the surface of the first substrate 210. That is, a pad part of the gas sensing element 100 and the metal pattern 220 of the first substrate are directly bonded by a flip chip bonding method so that a bonding wire can be removed, thereby enabling a reduction in a package area. Furthermore, a separate instrument, such as a cap member, a mesh member, and the like, is not required to be installed at an upper portion of the gas sensing part so that the package can be further miniaturized and a manufacturing cost can be reduced.

Also, the protective cap 30, which is an element intended for protecting the gas sensing element 100, is disposed to receive the gas sensing element 100 therein. Also, the protective cap 30 may be made of a metal material.

At this time, according to the present example, the protective cap 30 includes ventilation holes 35, 36, and as illustrated in FIGS. 1 and 2, the first ventilation hole 35 may be formed on the protective cap 30 to be in contact the first substrate 210. In such a case, the ventilation holes 35, 36 may be formed at the protective cap by selectively performing etching via a photo lithography process. The first ventilation hole 35 enables ventilation for allowing a reaction gas to sufficiently flow into the gas sensing element 100. As illustrated in FIG. 3, the first ventilation hole 35 is formed symmetrically based on the gas sensing element 100, thereby enabling efficient inflow of the reaction gas.

Also, as illustrated in FIG. 2, the second ventilation hole 36 is formed in an upper surface portion (32) of the protective cap 30. The second ventilation hole 36 may discharge the reaction gas entered into the protective cap 30. More specifically, according to a conventional bonding wire method, a protective cap having a higher height than that of the protective cap according to the flip chip bonding method should be used due to a height of a bonding wire. Furthermore, according to the flip chip bonding method, the height of the protective cap may be formed to be identical to a height of the element, and due to this, the problems of inflow and discharge of the reaction gas are generated.

Accordingly, according to the present example, the reaction gas may sufficiently flow into the gas sensing element 100 of the protective cap 30 via the first ventilation hole 35 and the reaction gas in the protective cap 30 may be discharged via the second ventilation hole 36 so that efficiency ventilation can be performed. Thus, the problems of ventilation and radiant heat, which may be generated due to a structural problem of the flip chip bonding method, can be simultaneously solved.

Also, the protective cap 30 may be made of a metal material so that the gas sensing element can be prevented from being damaged due to an external shock, and an external impact due to heat or pressure can be minimized. The metal material may be composed of any one of Al, SUS (Steel Use Stainless), and Cu, or may be composed of an alloy material in which any one element of Zn, Sn, Fe, Ni, Mn, Al, Si, and Pb is included in Al or Cu.

Also, the protective cap 30 according to the present example may further include an insulating surface treatment layer formed on a surface thereof.

More specifically, the surface treatment layer is formed on the surface of the protective cap 30 by anodizing so that the protective cap 30 can be prevented from causing a short circuit with the gas sensing element, an NTC (Negative Temperature Coefficient) thermistor, a resistance element, a terminal, or the like.

Also, according to the present example, as illustrated in FIGS. 2 and 3, on the substrate 210, an output change part 25, as well as the gas sensing element, are further included. The output change part 25 may be mounted using the flip chip bonding method.

The output change part 25 is electrically connected to the gas sensing element 10, thereby changing an output mode of the gas sensing element 100.

The output change part 25 may be composed of a passive element intended for converting the output of a resistance mode to the output of a voltage mode. A metal pattern and a fixed resistance or NTC (Negative Temperature Coefficient) thermistor may be used as the passive element.

By packaging a resistance output mode of the gas sensing element 120 as a voltage output mode, the output change part 25 may be applied to various IT devices (including a smart phone and the like). That is, a fixed resistance or NTC thermistor is applied to a side of an end of the gas sensing element 100 so that the resistance output mode can be converted into the voltage output mode.

In such a case, when the NTC thermistor is connected to the side of the end of the gas sensing element, it is advantageous in that a fixed initial voltage value can be obtained by compensating a resistance change value for an initial sensing material according to each temperature.

That is, when a resistance or NTC thermistor is used at the exterior of a printed circuit board, it is problematic in that an entire size of a module is increased, and a circuit design should be separately performed. However, when the package is implemented such that the output change part capable of converting the output mode of the gas sensor into the voltage mode is included in the inside of the package, the miniaturized gas sensor can be provided, and when a resistance change value of the NTC thermistor for each temperature is adopted at the same ratio as a resistance curve of the sensing material, temperature compensation can be performed, so initial resistance of the sensing material resulting from a temperature change can be also compensated.

Like the gas sensing element 100, the output change part 25 configured as described above may be formed on the first substrate 210 and may be configured to be covered by the protective cap 30.

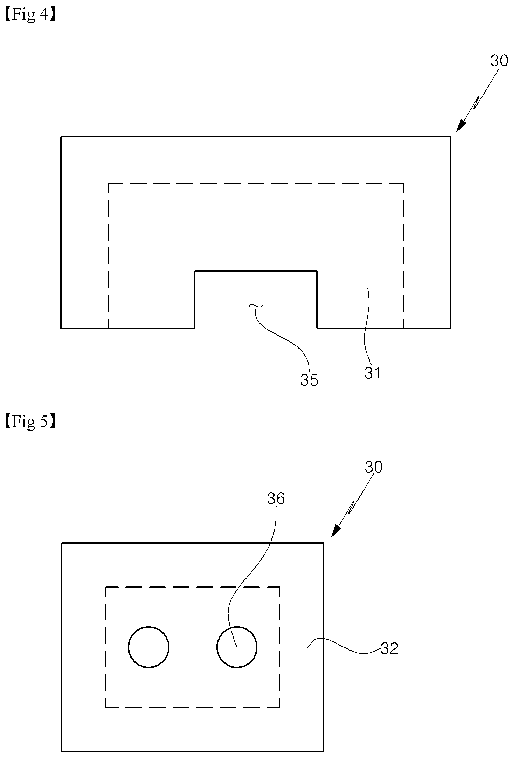

FIGS. 4 and 5 are views illustrating a protective cap according to one example. That is, FIG. 4 is a side view of the protective cap according to the one example, and FIG. 5 is a top view of the protective cap according to the one example.

The protective cap 30 according to one example is configured such that, as illustrated in FIG. 4, the first ventilation hole 35 is formed in a side wall 31 of the protective cap, and as illustrated in FIG. 5, the second ventilation hole 36 is formed in an upper surface portion 32.

The first ventilation hole 35 enables ventilation for allowing the reaction gas to sufficiently flow into the gas sensing element 100, and the second ventilation hole 36, formed in the upper surface portion of the protective cap 30, efficiently discharges the reaction gas entered into the protective cap 30.

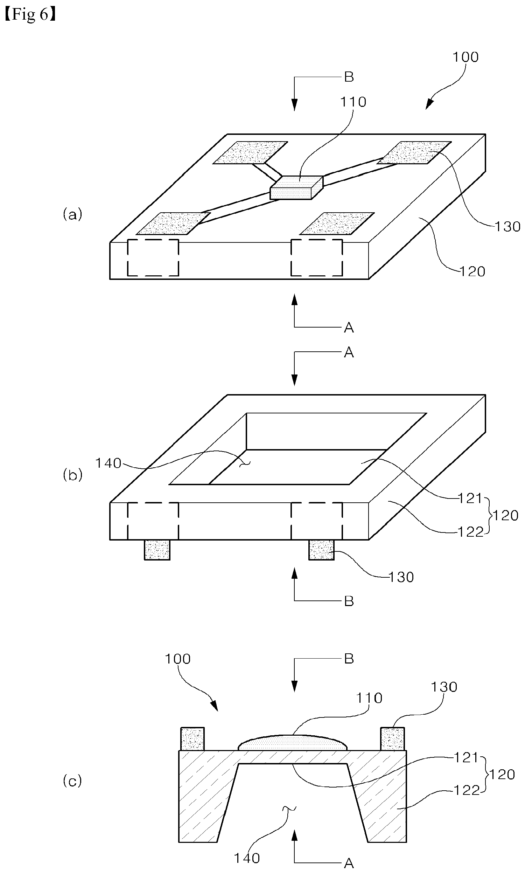

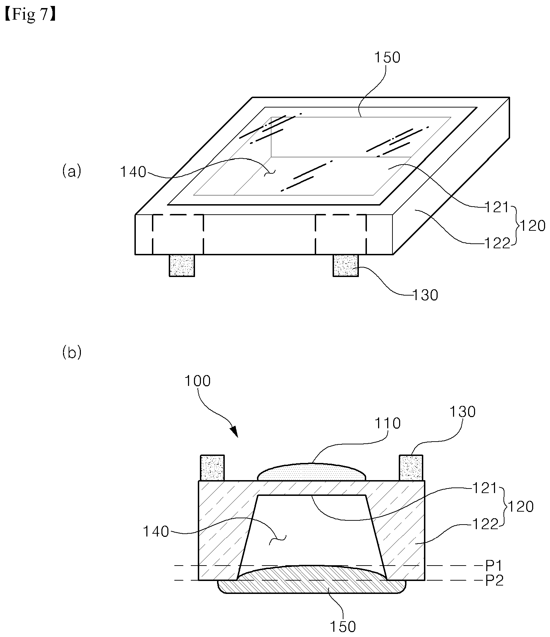

FIGS. 6 and 7 are the conceptual views of main subject matters illustrating a structure of the gas sensing element 100 applied to the embodiments of the present invention.

Referring to FIG. 6, the gas sensing element applied to the gas sensor package according to the example includes: an element body 120 including a lower surface 121 and a side wall 122 and having a cavity part 140 in the inside thereof; a cover member 150 for sealing an upper surface of the cavity part 140; and the gas sensing part 110 disposed on an external surface of the lower surface.

Specifically, (a) of FIG. 6 defines a lower direction A and an upper direction B of the gas sensing element of the present example and is a conceptual view of the main subject matters as viewed from the upper direction A; (b) of FIG. 6 is a conceptual view of the main subject matters as viewed from the lower direction A; and (c) of FIG. 6 is a side cross-sectional concept view.

Referring to FIG. 6, as shown in (a) of FIG. 6, when viewed from the upper directions B of the gas sensing element, the gas sensing part 110 detecting gas via a sensing material or a sensing chip is disposed on the external surface of the lower surface of the element body 120; and electrode patterns 130 connectable to an external terminal are provided on each adjacent surface, wherein the gas sensing part 110 and the electrode patterns 130 may be electrically connected to each other.

As shown in (b) of FIG. 6, when viewed from the lower direction B of the gas sensing element, the gas sensing element according to the present example is configured such that a side wall 122 and a lower surface 121 are implemented to form a cavity part 140 in the inside and is also configured to implement a region that is opened in the upper direction.

As shown in (c) of FIG. 6, when viewed based on the side cross-sectional view, the gas sensing part 110 is disposed on a surface opposite to the lower surface 121 of the element body having the cavity part 140 in the inside thereof, and the electrode patterns 130 are provided around the gas sensing part so as to be mounted on the substrate later.

FIG. 7 illustrates a structure of the gas sensing element described based on FIG. 6, in particular, a structure in which the cover member 150 for sealing the upper surface of the cavity part is implemented.

The cover member 150 is disposed to cover an upper surface of the side wall 122 and the upper surface of the cavity part of the element body and may be implemented in a structure in which a separate structure is processed and a synthetic resin film such as polyimide and the like is bonded to the structure. However, in the preferred embodiment of the present invention, the structure of the cover member is implemented to enable sealing by dropping a synthetic resin material having a fixed viscosity on the upper surface of the cavity part using a dispensing method, so that the inside of the cavity part and the gas sensing part can be protected, a space intended for gas stay for gas sensing can be secured in the gas sensing part, and a function of preventing radiant heat can be implemented. In particular, in the present example, the cover member 150 is intended to extensively fix an adjacent element, as well as covering only the upper surface of the gas sensing element so that a bonding ability of the gas sensing element can be increased as described above.

The cover member 150 is configured to enable covering by dropping a potting material on an upper surface of the element adjacent to the upper surface of the gas sensing element using a dispensing method and the like. Accordingly, a lower surface PI of the cover member according to the present example is formed below an imaginary horizontal surface P2 formed by an upper surface of the side wall 122 of the element body. This is intended to implement the lower surface of the cover member so as to have a curvature according to deflection of the synthetic resin material having a fixed viscosity due to gravity or a surface tension. This structure may also complementarily function to improve sensing sensitivity by implementing a function of facilitating gas stay and circulation due to the curvature of the upper surface of the cavity part has the curvature. In order to improve sensing efficiency, fine gas moving holes may be formed on the lower surface of the element body. In addition to this, the fine gas moving holes may be also formed on a part of the cover member. Furthermore, the cover member may be implemented not to come into contact with a surface of the first substrate or may be implemented to come into contact with only a part of the surface so that a gas moving separation part formed in a side portion of the gas sensing element can be prevented from being covered.

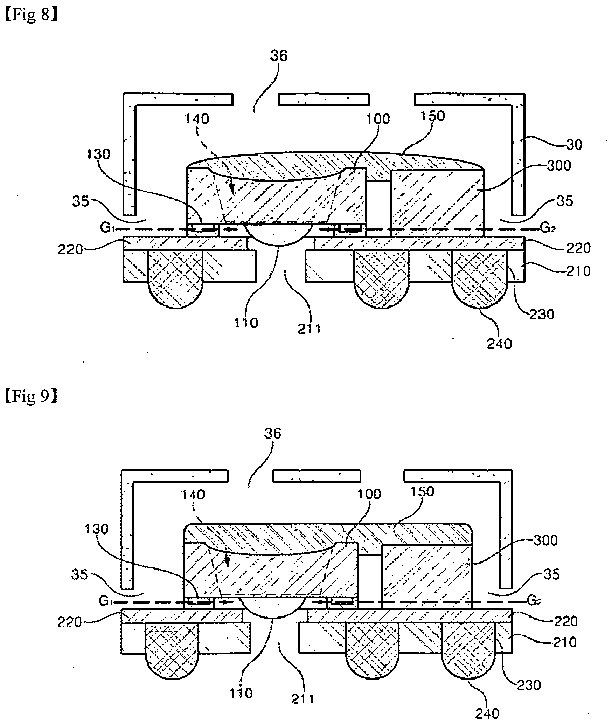

A gas sensor package will be hereinafter described with reference to FIGS. 8 and 9.

A structure shown in FIGS. 8 and 9 is identical to that of the previously described example, in that the gas sensing element 100 is mounted on the first substrate 210 in a flip chip structure, and the protective cap 30 is provided at an upper part thereof.

However, as illustrated in FIGS. 8 and 9, the gas sensing part 110 of the gas sensing element 100 may be aligned to correspond to a first through hole 211 of the first substrate 210 that may come into contact with gas according to the movement of external gas. This is because a structure in which the gas sensing part 110 is exposed via a gas inlet hole so that contact efficiency with gas can be increased, namely, a structure in which the gas sensing part 110 and a center portion of the gas inlet hole 211 are disposed to be aligned is the most efficient structure to increase sensing efficiency. Of course, the present invention is not limited to this structure, and the align configuration may be implemented such that the gas sensing part and the center portion of the gas inlet hole deviate from each other within a fixed scope. In such a case, according to the present embodiment of the invention, in terms of the gas sensing element, gas detection may be complemented by the gas moving separation parts G1, G2 and the like, so the same effect of improvement of the sensing efficiency can be implemented.

Moreover, at least one adjacent elements 300 mounted on the first substrate may be further included. Such an adjacent element may cover both an active element and a passive element. As one example, a fixed resistance element or NTC (Negative Temperature Coefficient) thermistor may be further included. In the case of the fixed resistance element or NTC (Negative Temperature Coefficient) thermistor, a resistance output mode is converted into a voltage output mode, in particular, in the case of the NTC thermistor, a resistance change value according to each temperature is compensated for an initial sensing material so that the NTC thermistor can have a regular initial voltage value.

In particular, it is more preferable to provide the cover member 150 intended for covering both the gas sensing element 100 and an upper portion of the adjacent element 300. The configuration of the cover member 150 is a special structure of the present invention and is intended to provide the cavity part 140 forming a gas stay region in the inside of the gas sensing element 100 and to control radiant heat while protecting the inside of the cavity part by sealing an upper portion of the cavity part. Furthermore, when the upper surface of the adjacent element is fixed by the cover member 150, in addition to protecting the gas sensing element, the adjacent element is also fixed so that a bonding ability of the gas sensing element itself can be improved. It has been described that the adjacent element is fixed by the cover member, but in addition to the adjacent element, chip structures such as an active element or a passive element, and structures (protruding structures except for the gas sensing element) of various chip packages may be fixed.

Various materials having an insulating property may be applied to the cover member 150. For example, in particular, a material including epoxy, urethane and Si as a synthetic resin material, a material used as a sealant and the like may be applied. It is preferable for this synthetic resin material to apply a material having a fixed viscosity so that the material cannot flow into the inside of the cavity part.

FIG. 8 shows a case in which a material having a relatively high viscosity is implemented as the cover member, and FIG. 9 shows a case in which a material having a low viscosity is implemented as the cover member. They show a difference in shapes of the cover member. In the case of FIG. 8, when epoxy is used as the material of the cover member, the material having a higher viscosity than that of the material of FIG. 9 is used so that the upper portion of the cover member can have a curvature, and the cover member may be implemented so that a lower portion can also have a curvature. According to the need, as shown in FIG. 2, epoxy having a lower viscosity than that of FIG. 1 is applied so that the upper portion of the cover member can be implemented in a flat structure, thereby enabling a slimming structure.

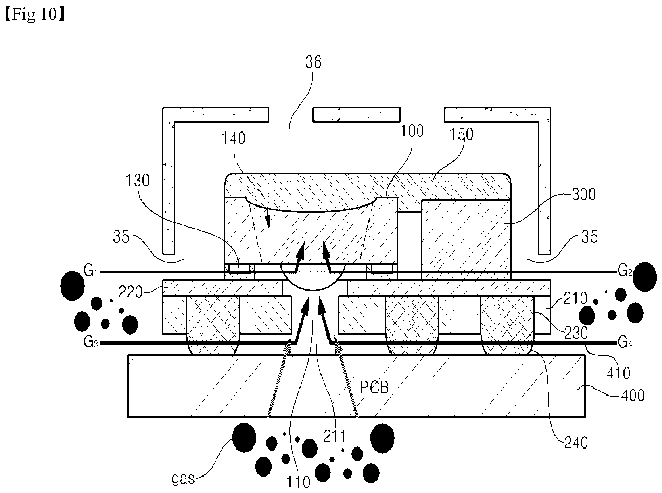

FIG. 10 is a conceptual view illustrating the structure of a gas sensor package according to another embodiment. The structure shown in the present embodiment is identical to that of the previously described gas sensor package, in that the gas sensing element 100 is mounted on the first substrate 210 in a flip chip structure, and the protective cap 30 is provided at an upper part thereof.

Referring to FIG. 10, the gas sensor package according to the present embodiment of the invention having the structure in which the gas sensing element 100 and the first substrate 210 are coupled to each other may be implemented to be mounted on a second substrate 400 such as a separate printed circuit board and the like.

The printed circuit board is applied as the second substrate 400. In particular, the printed circuit board may be made of a flexible material. As illustrated in FIG. 10, a metal filling part 240 of the first substrate 210 according to the present embodiment of the invention is electrically connected to the second substrate 400 corresponding to the printed circuit board. In particular, in such a case, the metal filling part 240 is configured to partially protrude in a direction of a lower surface of the first substrate 210 and has a fixed separation part even after coming into contact with the second substrate 400. As illustrated, the separation part forms gas moving paths G3, G4 (hereinafter referred to as 'the second gas moving separation parts').

The gas moving separation part 410 enables gas to be in direct contact with the gas sensing part 110 via the first through hole 211 provided in the first substrate in the gas sensor package according to the present embodiment of the invention and may also enable gas approaching from a side portion of the gas sensing element to be in contact with the gas sensing part, thereby ensuring an increase in sensing efficiency.

The conventional gas sensors are implemented in such a manner that the gas sensing part is disposed to face an upper surface of the substrate. This is intended to ensure contact efficiency with gas. Thus, a size of the package is necessarily increased because the gas sensing part should be disposed to face the upper portion and a protective net having a mesh structure should be formed. On the contrary, in the case of the gas sensor package according to the present embodiment of the invention, mounting is implemented so that a part where the gas sensing part is can be in contact with the surface of the first substrate, and a separate cap is not installed so that the package can be miniaturized, and a manufacturing cost can be reduced. Furthermore, gas stay using the cavity part formed in the inside of the element and the separation part intended for guiding gas from the side of the first through hole of the substrate to the gas sensing part are implemented so that sensing efficiency can be also secured.

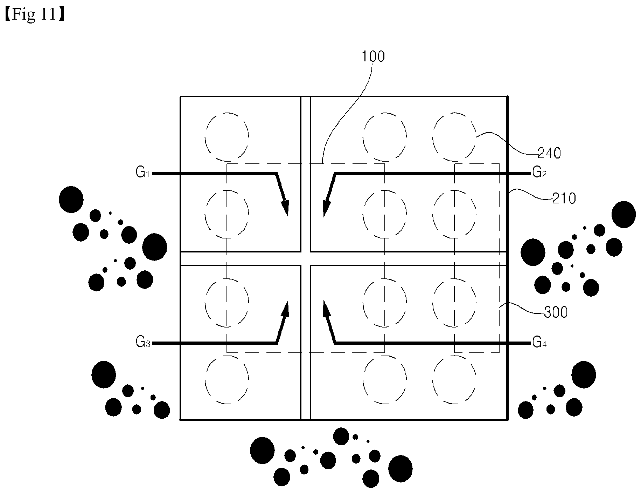

FIG. 11, which is a plan concept view of the gas sensor package as viewed from an upper portion of the gas sensor package in FIG. 10, illustrates moving paths G3, G4 for gas moving via the second gas moving separation parts after a lower PCB and the first substrate 210 have been combined with each other. As illustrated, the gas moving from the side of the gas sensing element 100 is transmitted via the gas moving separation parts G1, G2, and ventilation of the gas is smoothly performed via the second gas moving separation parts G3, G4 between the first substrate and the second substrate so that the gas can be transmitted to the gas sensing part.

As set forth above, according to some embodiments of the present invention, the gas sensing element and the substrate are directly bonded by a flip chip bonding method so that a bonding wire can be removed, a manufacturing cost can be reduced, and the gas sensor package can be miniaturized.

Also, according to some embodiments of the present invention, the first ventilation hole enables the reaction gas to sufficiently flow into the gas sensing element in the protective cap, and the second ventilation hole may discharge the reaction gas in the protective cap to enable efficient ventilation, so the problems of ventilation and radiant heat, which may be generated due to the structural problem of the flip chip bonding method, can be simultaneously solved.

As previously described, in the detailed description of the invention, having described the detailed exemplary embodiments of the invention, it should be apparent that modifications and variations can be made by persons skilled within the scope of the appended claims.

## Claims

1. A gas sensor package, comprising:
a first substrate (210) including a metal pattern (220);
a gas sensing element (100) mounted on the first substrate (210) and having a gas sensing part (110) disposed to face a surface of the first substrate (210);
and
a protective cap (30) disposed on the first substrate (210) and having a receiving space for protecting the gas sensing element (100),
wherein the protective cap (30) comprises a side wall (31) whose one end is in contact with the first substrate (210), an upper surface portion (32) on the side wall (31), and a first ventilation hole (35);
**characterised in that** said first ventilation hole (35) extends through the side wall (31) and connects the receiving space and an exterior, and
wherein the first ventilation hole (35) is in contact with the first substrate (210)
wherein the gas sensing element (100) comprises:
an element body (120) including a lower surface (121) and a side wall (122) and having a cavity part (140) in the inside thereof; and
the gas sensing part (110) is disposed on an external surface of said element body (120) opposite to said lower surface (121) facing said cavity part (140) and including a gas sensing material; and further **characterised by** comprising
a cover member (150) configured to cover an upper surface of the cavity part (140),
wherein the cover member (150) is disposed to cover an upper surface of the side wall (122) of the element body (120) and the upper surface of the cavity part (140) of the element body (120),
wherein a lower surface (P1) of the cover member (150) is formed below an imaginary horizontal surface (P2) formed by the upper surface of the side wall (122) of the element body(120), and has a curvature,
wherein an upper surface of the cover member (150) has a flat structure.

2. The gas sensor package of claim 1, further comprising a second ventilation hole (36) passing through the upper surface portion.

3. The gas sensor package of claim 1 or 2, wherein the protective cap (30) is made of a metal material.

4. The gas sensor package of claim 3, wherein the metal material is composed of any one of Al, and Cu, or is composed of an alloy material in which any one element of Zn, Sn, Fe, Ni, Mn, Al, Si, and Pb is included in Al or Cu.

5. The gas sensor package of any one of claims 1 to 4, comprising an insulating surface treatment layer provided on a surface of the protective cap (30).

6. The gas sensor package of claim 5, wherein the insulating surface treatment layer is an anodizing layer implemented on the surface of the protective cap (30).

7. The gas sensor package of any one of claims 1 to 6, wherein the first substrate (210) comprises a gas inlet hole (211) passing through the first substrate (210).

8. The gas sensor package of claim 7, wherein the gas inlet hole (211) and the gas sensing part (110) are aligned to correspond to each other.

9. The gas sensor package of any one of claims 1 to 8, wherein the first substrate (210) further comprises at least one through hole passing through a lower portion of the metal pattern on the first substrate (210).

10. The gas sensor package of claim 9, further comprising a metal filling part (240) filled in the through hole.

11. The gas sensor package of claim 10, wherein the metal filling part (240) protrudes to the exterior of a lower surface of the first substrate (210).

12. The gas sensor package of claim 10 or 11, further comprising a second substrate (400) connected to a lower portion of the first substrate (210) via the metal filling part (240).

## Patentansprüche

1. Gassensorpaket, umfassend:
ein erstes Substrat (210) umfassend ein Metallmuster (220);
ein Gassensorelement (100), das an dem ersten Substrat (210) montiert ist und ein Gassensorteil (110) aufweist, das so angeordnet ist, dass es einer Oberfläche des ersten Substrats (210) zugewandt ist;
und
eine Schutzkappe (30), die an dem ersten Substrat (210) angeordnet ist und einen Aufnahmeraum zum Schützen des Gassensorelements (100) aufweist,
wobei die Schutzkappe (30) eine Seitenwand (31), deren eines Ende in Kontakt mit dem ersten Substrat (210) ist, einen oberen Oberflächenabschnitt (32) an der Seitenwand (31), und ein erstes Lüftungsloch (35) umfasst;
**dadurch gekennzeichnet, dass** sich das erste Lüftungsloch (35) durch die Seitenwand (31) erstreckt und den Aufnahmeraum und eine Außenseite verbindet, und
wobei das erste Lüftungsloch (35) in Kontkat mit dem ersten Substrat (210) ist,
wobei das Gassensorelement (100) umfasst:
einen Elementkörper (120), der eine untere Oberfläche (121) und eine Seitenwand (122) umfasst und ein Hohlteil (140) im Inneren von diesem aufweist; und
wobei das Gassensorteil (110) an einer äußeren Oberfläche des Elementkörpers (120) gegenüber der dem Hohlteil (140) zugewandten unteren Oberfläche (121) angeordnet ist und ein Gassensormaterial umfasst; und
ferner **dadurch gekennzeichnet, dass** es umfasst
ein Abdeckelement (150), das dazu konfiguriert ist, eine obere Oberfläche des Hohlteils (140) abzudecken,
wobei das Abdeckelement (150) so angeordnet ist, dass es eine obere Oberfläche der Seitenwand (122) des Elementkörpers (120) und die obere Oberfläche des Hohlteils (140) des Elementkörpers (120) abdeckt,
wobei eine untere Oberfläche (P1) des Abdeckelements (150) unter einer gedachten horizontalen Oberfläche (P2) gebildet ist, die durch die obere Oberfläche der Seitenwand (122) des Elementkörpers (120) gebildet ist, und eine Krümmung aufweist,
wobei eine obere Oberfläche des Abdeckelements (150) eine flache Struktur aufweist.

2. Gassensorpaket nach Anspruch 1, ferner umfassend ein zweites Lüftungsloch (36), das durch den oberen Oberflächenabschnitt hindurchgeht.

3. Gassensorpaket nach Anspruch 1 oder 2, wobei die Schutzkappe (30) aus einem Metallmaterial hergestellt ist.

4. Gassensorpaket nach Anspruch 3, wobei das Metallmaterial aus irgendeinem von Al und Cu besteht, oder aus einem Legierungsmaterial besteht, bei dem irgendein Element aus Zn, Sn, Fe, Ni, Mn, Al, Si und Pb in Al oder Cu enthalten ist.

5. Gassensorpaket nach einem der Ansprüche 1 bis 4, umfassend eine Isolieroberflächenbehandlungsschicht, die an einer Oberfläche der Schutzkappe (30) vorgesehen ist.

6. Gassensorpaket nach Anspruch 5, wobei die Isolieroberflächenbehandlungsschicht eine Anodisierschicht ist, die an der Oberfläche der Schutzkappe (30) implementiert ist.

7. Gassensorpaket nach einem der Ansprüche 1 bis 6, wobei das erste Substrat (210) ein Gaseinlassloch (211) umfasst, das durch das erste Substrat (210) hindurchgeht.

8. Gassensorpaket nach Anspruch 7, wobei das Gaseinlassloch (211) und das Gassensorteil (110) einander entsprechend fluchtend ausgerichtet sind.

9. Gassensorpaket nach einem der Ansprüche 1 bis 8, wobei das erste Substrat (210) ferner wenigstens ein Durchgangsloch umfasst, das durch einen unteren Abschnitt des Metallmusters an dem ersten Substrat (210) hindurchgeht.

10. Gassensorpaket nach Anspruch 9, ferner umfassend ein Metallfüllteil (240), das in das Durchgangsloch gefüllt ist.

11. Gassensorpaket nach Anspruch 10, wobei das Metallfüllteil (240) zur Außenseite einer unteren Oberfläche des ersten Substrats (210) vorsteht.

12. Gassensorpaket nach Anspruch 10 oder 11, ferner umfassend ein zweites Substrat (400), das mit einem unteren Abschnitt des ersten Substrats (210) über das Metallfüllteil (240) verbunden ist.

## Revendications

1. Un boîtier pour capteur de gaz, comprenant :
un premier substrat (210) comprenant une structure métallique (220) ;
un élément (100) de détection de gaz monté sur le premier substrat (210) et ayant une partie (110) de détection de gaz disposée de façon à être tournée vers une surface du premier substrat (210) ; et
un capot protecteur (30) disposé sur le premier substrat (210) et ayant un espace de réception pour protéger l'élément (100) de détection de gaz,
le capot protecteur (30) comprenant une paroi latérale (31) dont une extrémité est en contact avec le premier substrat (210), une partie (32) formant surface supérieure située sur la paroi latérale (31) et un premier trou de ventilation (35) ;
**caractérisé en ce que** ledit premier trou de ventilation (35) s'étend à travers la paroi latérale (31) et relie l'espace de réception et l'extérieur, et
dans lequel le premier trou de ventilation (35) est en contact avec le premier substrat (210),
l'élément détecteur de gaz (100) comprenant :
un corps d'élément (120) comprenant une surface inférieure (121) et une paroi latérale (122) et ayant une partie (140) formant cavité à l'intérieur de lui ; et
la partie (110) de détection de gaz est disposée sur une surface externe dudit corps d'élément (120) opposée à ladite surface inférieure (121) tournée vers ladite partie (140) formant cavité et comprenant un matériau de détection de gaz ; et **caractérisé en outre en ce qu'**il comprend
un élément de recouvrement (150) configuré pour recouvrir une surface supérieure de la partie (140) formant cavité,
l'élément de recouvrement (150) étant disposé de façon à recouvrir une surface supérieure de la paroi latérale (122) du corps de l'élément (120) et la surface supérieure de la partie (140) formant cavité du corps de l'élément (120),
une surface inférieure (P1) de l'élément de recouvrement (150) étant formée au-dessous d'une surface horizontale imaginaire (P2) formée par la surface supérieure de la paroi latérale (122) du corps de l'élément (120), et ayant une courbure,
une surface supérieure de l'élément de recouvrement (150) ayant une structure plate.

2. Le boîtier de capteur de gaz selon la revendication 1, comprenant en outre un deuxième trou de ventilation (36) traversant la partie formant la surface supérieure.

3. Le boîtier de capteur de gaz selon la revendication 1 ou la revendication 2, dans lequel le capot protecteur (30) est réalisé en un matériau métallique.

4. Le boîtier de capteur de gaz selon la revendication 3, dans lequel le matériau métallique est composé de l'un quelconque parmi Al et de Cu, ou est composé d'un matériau d'alliage dans lequel un élément quelconque de Zn, Sn, Fe, Ni, Mn, Al, Si et Pb est compris dans Al ou Cu.

5. Le boîtier de capteur de gaz selon l'une quelconque des revendications 1 à 4, comprenant une couche de traitement de surface isolante prévue sur une surface du capot protecteur (30).

6. Le boîtier de capteur de gaz selon la revendication 5, dans lequel la couche de traitement de surface isolante est une couche d'anodisation appliquée sur la surface du capot protecteur (30).

7. Le boîtier de capteur de gaz selon l'une quelconque des revendications 1 à 6, dans lequel le premier substrat (210) comprend un trou d'entrée de gaz (211) traversant le premier substrat (210).

8. Le boîtier de capteur de gaz selon la revendication 7, dans lequel le trou d'entrée de gaz (211) et la partie (110) de détection de gaz sont alignés pour correspondre l'un à l'autre.

9. Le boîtier de capteur de gaz selon l'une quelconque des revendications 1 à 8, dans lequel le premier substrat (210) comprend en outre au moins un trou traversant qui traverse une partie inférieure de la structure métallique sur le premier substrat (210).

10. Le boîtier de capteur de gaz selon la revendication 9, comprenant en outre une partie métallique de remplissage (240) placée dans le trou traversant de façon à remplir ce dernier.

11. Le boîtier de capteur de gaz selon la revendication 10, dans lequel la partie métallique de remplissage (240) fait saillie vers l'extérieur d'une surface inférieure du premier substrat (210).

12. Le boîtier de capteur de gaz selon la revendication 10 ou la revendication 11, comprenant en outre un deuxième substrat (400) relié à une partie inférieure du premier substrat (210) via la partie métallique de remplissage (240).
